# EUROPEAN PATENT APPLICATION

(11) **EP 4 576 121 A1**
(43) Date of publication of application: **25.06.2025**
(21) Application number: 23219109.8
(22) Date of filing: 21.12.2023
(51) Int. Cl.: G16H 80/00, G10L 25/63, G06N 20/00

(54) **SUPPORT SYSTEM FOR ACTIVE LISTENING IN CONVERSATION SITUATIONS**

(71) Applicant: HONDA MOTOR CO., LTD., Minato-ku Tokyo 107-8556 (JP)
(72) Inventor: Fischer, Lydia, 63073 Offenbach/Main (DE); Weisswange,Thomas, 63073 Offenbach am Main (DE)
(74) Representative: Beder, Jens

(57) **Abstract**

The disclosure regards a system, method and program for assisting a person in improving its conversation skills. The system comprises a perception module configured to perceive a conversation between the person and at least one other person by generating information representing the conversation based on acquired sensor information, and a processing module configured to classify the assisted person and the at least one other person into at least one listener and a speaker based on the generated information. The processing module is further configured to determine which potential behavior of the person classified as the listener correlates with a positive quality of the conversation by evaluating the current conversation situation based on the generated information, to determine which actual behavior the person classified as the listener is performing based on the generated information, and to determine at least one additional behavior for execution by the person classified as the listener that improves the quality of the conversation based on the generated information. The system comprises an interface configured to output a recommendation for an improved communication behavior to the person classified as the listener generated based on the determined at least one additional behavior.

## Description

The disclosure in the field of human communication and in particular concerns systems and methods for analyzing conversation between humans and assisting humans in improving their communication skills.

Humans communicate verbally via speech and non-verbally. For achieving a high quality communication between people, it is important to attend to the verbally communicated information and simultaneously to perceive the non-verbal information. This is particularly important in situations including a one-on-one communication between two humans.

The quality of communication not only depends on speaking skills, but also on listening skills. In professional communication situations, e.g., between a doctor and a patient, between a lawyer and his client, between a nurse and a person in need of care, the communication quality is often addressed via the target of optimizing a solution to a problem, and not by providing a positive feeling for the participants in the communication. Consequentially, the listening behavior often focusses on a correct understanding of facts but ignore the target of returning the feeling of being heard to the current speaker. Improving listening skills can be practiced with human experts, but these can hardly give feedback in real situations due to issues of availability of the human experts, privacy of the communicated information.

Psychologists Carl Rogers and Richard Farson Rogers in "Active listening, Industrial Relations Center of the University of Chicago, 1957, defined the concept of active listening. They describe the skill of active listening as vitally important for effective communication. For Carl Rogers, the ultimate goal of active listening was to foster positive change.

There exist communication support systems that support a listening person (listener) in order to obtain the facts mentioned in the conversation, but they do not support in terms of an emotional context that the speaking person (speaker) is obtains a feeling of being heard by the listener, is understood, or being trusted. A lack thereof may result in a communication or a relationship between the speaker and the listener that lacks trust between the participants, potentially causing a hiding of important facts .

Currently approaches exist that support improving speaking skills, e.g., by analyzing a speaking behavior and providing suggestions how to improve speaking skills, both with respect to content and to conveying a positive image to the listener. However, listening skills differ from speaking skills.

There exists social robot technologies, which use a limited number of listening behaviors widely considered as good listening behavior to improve an emotional quality of conversations with humans.

Moreover, an analysis of conversational behaviors presently is regularly limited to the features of one participant in a communication at a time.

Current approaches also focus on communication scenarios that involve video call. However, a physical face-to-face setup influences the human communication significantly, which approaches of analyzing conversational behaviors can only partly grasp.

US 9,131,053, B1 discloses a method for improving a call-participant behavior ba receiving an intensity data signal and an intensity variation data signal related to an ongoing call, receiving a pitch data signal and a pitch variation data signal related to the ongoing call, receiving a tempo data signal and a tempo variation data signal related to the ongoing call, and receiving a channel comparison data signal related to the ongoing call. A real-time call progress signal is generated based on the received signals, and the real-time call progress signal is sent to a user device.

US 6,959,079 B2 describes: a monitoring system for monitoring agent telephonic interactions with customers and a method thereof is provided. The system may comprise a voice logger to receive and record audio of a telephone call received by the agent, a screen logger to receive and record video screen data associated with interactions of the agent with a computer during the telephone call and an event manager to determine whether the interactions meet at least one predefined monitoring condition.

US 2014/0314212 A1 suggests a system monitoring a conversation, analyzing the (foreground) speech and (background) respiratory sounds, and displaying to the call-taker or dispatcher any parameters detected outside of normal limits or beyond set thresholds. In some embodiments, an analysis of the breathing and speech of the speaker may be performed on background sounds in the same room as a caller who is not a patient.

US 1,122,8731 B1 relates to methods and systems for providing an artificial intelligent assistant to increase the effectiveness of communications. The method comprises receiving an audio-visual electronic communication at an artificial intelligence assistant computing platform from a first user, the audio-visual electronic communication comprising an audio communication content and a video communication content. The intended recipient is a second user. Audio communication information and video communication information is extracted from the audio and video communication content, respectively. A compositional change for the communication content of the audio-visual electronic communication is generated based on non-verbal information determined from the extracted audio or video communication information and a changed electronic communication is generated from the audio-visual electronic communication and the compositional change by directly making at least one change to the audio-visual electronic communication. The changed electronic communication is a modified version of the audio-visual electronic communication. The at least one change includes a style transformation that is a vocabulary shift.

Considering the aforementioned aspects, there remains the task of improving the state of the art by providing solutions for analyze data from a conversation with respect to listening behaviors and indicators for speaker satisfaction and to support to participants in the conversation for improving their listening skills.

The system for assisting a person in improving its conversation skills according to claim 1, the computer-implemented method and the computer program according to the corresponding independent claims address the problem.

The dependent claims define further advantageous embodiments.

The system for assisting a person in improving its conversation skills in a first aspect of the disclosure includes a perception module configured to perceive a conversation between the person and at least one other person by generating information representing the conversation based on acquired sensor information and a processing module configured to classify the assisted person and the at least one other person into at least one listener and a speaker based on the generated information. The processing module is further configured to determine, which potential behavior of the person classified as the listener correlates with a positive quality of the conversation by evaluating the current conversation situation based on the generated information, to determine which actual behavior the person classified as the listener is performing based on the generated information, and to determine at least one additional behavior for execution by the person classified as the listener that improves the quality of the conversation based on the generated information. An interface is configured to output a recommendation for an improved communication behavior to the person classified as the listener generated based on the determined at least one additional behavior.

The system uses known image recognition and speech recognition techniques for determining specific listening behaviors of the participants in a conversation. The system correlates detected behaviors with an evaluation of speaker satisfaction, detects opportunities for a positive or active listening behavior in situations when speaker satisfaction is determined to decrease. The system is able to provide feedback to the listener that supports the listener in improving his listening behavior, which in turn also influences advantageously the communication quality of the conversation and the emotional state of the speaker.

The approach implemented in the system explicitly extracts and models listening behaviors of the participants in the conversation, contrary to the known approaches that emphasize the classic speaker qualities.

The system relies on an analysis of situational features between both participants in order to evaluate the listening quality and the communication quality (conversation quality). The system is capable to determine a difference between all the potential behaviors of the listener in a current situation in the conversation scenario, and the actual behaviors that the listener actually executes in the current situation in the conversation scenario. This enables to identify additional behaviors from the potential behaviors of the listener, that in the current situation correlate with a positive quality of the listening behavior, which the listener is not executing in the current situation, but which the system deems nevertheless suitable to improve a quality of the listening behavior towards the target of active listening.

The system estimates an actual satisfaction of the speaker as a target indicator for the communication quality and can therefore dispense with using out-of-situation labels.

The system enables using a multimodal interface to the listener for the system that may even include visual information presented on a screen, e.g., a computer display, a tablet display, or a smartphone display, and acoustic information output via an in-ear speaker. The system may also use tactile signals, e.g., vibration actuators as part of the multimodal human machine interface.

The system enables a dynamic switching of roles of the speaker and the listener between the participants in the conversation by a dynamic assignment of the speaking role and the listening role during the course of the conversation.

The system achieves its target is to increase emotional satisfaction of the other participant in the conversation with the assisted person.

The system has the advantageous effect of enhancing an active listening performance during a conversation and across plural conversations.

The system is advantageous due to achieving its effects without requiring to extract any communication contents and semantics, and thereby operates in a privacy-preserving way.

According to an embodiment of the system, the perception module is configured to generate the information representing the conversation based on the perceived complete conversation of the person and the at least one other person; the processing module is configured to analyze the information offline, and the interface is configured to output the recommendation for an improved communication behavior to the person classified as the listener generated based on the determined at least one additional behavior for the complete conversation as training information.

The system according to an embodiment includes the perception module configured to generate the information representing the conversation based on the perceived conversation of the person and at least one other person while the conversation is ongoing; the processing module is configured to analyze the information online while the conversation is ongoing, and the interface is configured to output the recommendation for an improved communication behavior to the person classified as the listener generated based on the determined at least one additional behavior as an online guidance while the conversation is ongoing.

In an embodiment of the system, the processing module is configured to determine based on the information representing the conversation a satisfaction measure for the person classified as the speaker with the conversation.

The system according to an embodiment includes the processing module configured to dynamically reclassify the person and the at least one other person into a t least one new listener and a new speaker based on the generated information representing the conversation during the conversation.

According to an embodiment of the system, the processing module is configured to control the interface output the recommendation for an improved communication behavior to the person classified as the at least one new listener after dynamically reclassifying the person and the at least one other person into at least one new listener and a new speaker.

The system according to an embodiment comprises a privacy filter configured to filter at least one of the acquired sensor information and the generated information representing the conversation by applying at least one predetermined confidentiality rule on the at least one of the acquired sensor information and the generated information representing the conversation.

A computer-implemented method for assisting a person in improving its conversation skills in a second aspect of the disclosure comprises a step of perceiving, by a perception module, a conversation of the person and at least one other person by generating information representing the conversation based on acquired sensor information. The method proceeds with classifying, by a processing module, the assisted person and the at least one other person into at least one listener and a speaker based on the generated information and determining, by the processing module, which potential behavior of the person classified as the listener correlates with a positive quality of the conversation by evaluating the current conversation situation based on the generated information. The method determines, by the processing module, which actual behavior the person classified as the listener is performing based on the generated information, and also determines, by the processing module, at least one additional behavior for execution by the person classified as the listener that improve the quality of the conversation based on the generated information. The method then performs a step of controlling, by the processing module, an output via an interface to the person classified as the listener of a recommendation for an improved communication generated based on the determined at least one additional behavior.

A computer program according to a third aspect of the disclosure comprises instructions, which, when a computer or signal processor executes the computer program, causes the computer or signal processor to carry out the method according to the second aspect.

The following description of embodiments refers to the figures, in which
Fig. 1 provides an overview of the system and its major components in an embodiment;
Fig. 2 presents an overview of the architecture of the behavior evaluation by an embodiment of the system;
Fig. 3 presents an example of a screen of the interface illustrating an aspect of the architecture of an embodiment of the system;
Fig. 4 illustrates an example design of an interface device of the system in an embodiment; and
Fig. 5 shows a schematic flow diagram illustrating steps of a computer-implemented method according to an embodiment.

In the figures, corresponding elements have the same reference signs. The discussion of the figures avoids discussion of same reference signs in different figures wherever considered possible without adversely affecting comprehensibility and avoiding unnecessary repetitions for sake of conciseness.

The disclosure concerns human communication, in particular conversation between humans, and assisting humans in improving their communication skills in a conversation with other humans.

During a conversation, the participants in the conversation have certain roles for a given amount of time while the conversation is ongoing. Usually, a participant (person) that wants to share information or requests an issue being solved has the role of a speaker. The speaker initializes a conversation and the topic of the conversation, usually has a higher amount of speaking time, and has specific targets that the conversation is intended to reach from the speaker's point of view. A participant in the conversation that has the role of a listener may try to support the speaker by responding to requests and by analyzing information disclosed in the conversation. The listener usually has less speaking time and less control over topic than the speaker has. The listener is required to respond to content presented by the speaker. Each participant will share the additional target of feeling treated well and valued by the other participant.

One might refer to the person that is receiving the information to improve its listening behavior as a user, while other persons participating in a communication, who not receive system output for supporting their conversation skills, are referred to as non-users. The user might often also be the owner of the system. There exists the possibility that multiple participants are considered as users, e.g., in embodiments and scenarios in which the system switches an addressee of the supporting information dynamically during the conversation, e.g., in cases in which a change in the assignment of the listener role and the speaker role occur.

The disclosure uses the term quality of a communication or communication quality. The communication quality is determined by the individual evaluations of the participants at any moment or at the end of a conversation. The communication quality usually includes the success of exchanging facts, the solution of conversation tasks, the occurrence of emphatic moments in the communication, and each participant feeling heard, understood, and valued. Assessing the quality of the communication for an individual participant may prove difficult as assessing the communication quality often includes internal evaluations of the other participant. The indicator or measure speaker satisfaction refers to the communication quality as evaluated by the participant that has currently the speaker role and corresponds to the fulfillment of the conversational targets of the speaker.

Active listening is a communication skill that involves going beyond the participant having the listening role simply hearing the words that another person speaks but also seeking to understand the meaning of the words and an intent associated with the conversation. It requires being an active participant in the communication process. Active listening may involve a plurality of behaviors that can be performed during a conversation, including paraphrasing, using open questions or asking exploring questions, reflecting on emotions, acknowledging non-verbally the perceived speech, providing feedback (back-channeling), summarizing understood content, presenting an open body posture and maintaining eye-contact with the speaker. The present disclosure includes examples for positive listening behaviors that extend beyond the original concepts of active listening, but are subsumed under this term in the present disclosure.

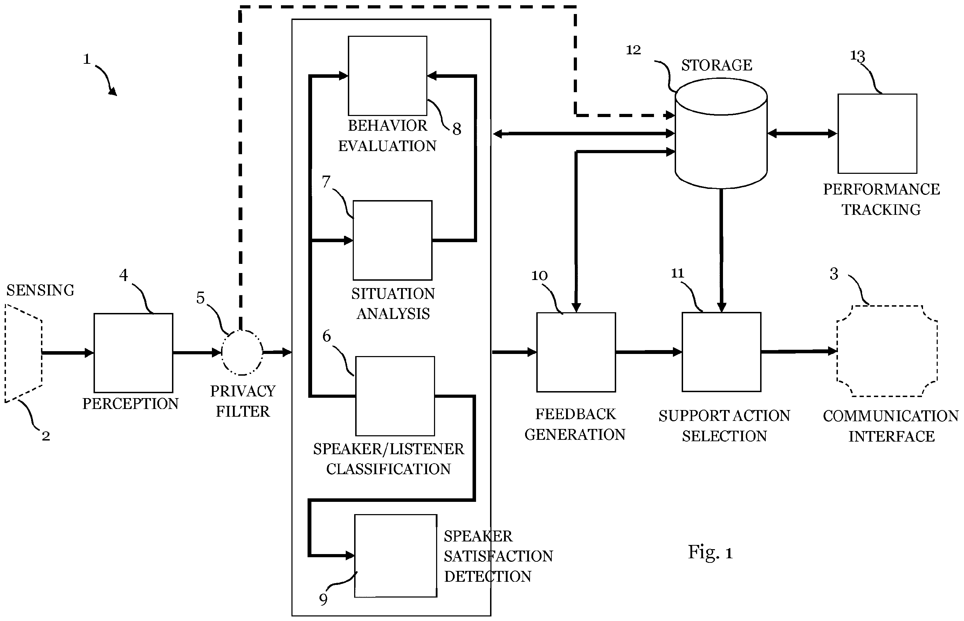

Fig. 1 provides an overview of the system 1 and its major components in an embodiment.

The system 1 acquire from at least one of external sensing means 2 and own sensing means 2 of the system 1 information on the environment in which at least two persons participating in a conversation are present.

The system1 comprises at least one computing module (processing module) that obtains the information on the environment that describes a situation in the environment as sensed, processes the information on the environment, extracts further information from the obtained information and provides results of a computation to an output interface device 3 that communicates the results to a user of the system 1.

The sensing means 2 includes at least one sensor for acquiring sensor data of the user and at least one other person during a conversation between the user and the at least one other person.

The at least one sensor may be part of a device including the components of the system 1. Alternatively, the at least one sensor may be part of one or more individual devices that are installed or positioned in the environment, which are connected to the computing module and transmit the information acquired from the environment as data via a communication means to the computing module. The communication means may include a wire-based, an optical fiber-based or a wireless communication link. The wireless communication link may include at least one of a WLAN, a WPAN, a Bluetooth link or a mobile radio link.

The sensors may include any combination of at least one camera, microphone, pressure sensor, e.g. positioned in the floor or on a chair, eye-tracker, wearable sensor for locating bodies of humans in the room or limbs of humans relative to each other, EEG, heat or infrared sensors, and even chemical sensor, e.g., for detecting sweat.

The sensors generate and output sensor data that may include at least one of video data, image data, and audio data. The sensor data may include pressure signal data, gaze data, brain activity data, body state data, signal time series data, muscle tension data, in order to name at least some examples.

The sensors provide the sensor data including the information on the environment to a perception module 4 for an analysis, in which the perception module extracts information using analysis methods that may include machine learning, image processing or signal processing or a combination thereof.

The perception module 4 extracts perception information that may include one or more of characteristic information of the user and the at least one other person, including information on at least one of body posture, facial expression, gaze direction, relative position of persons, activities of the persons, micro-actions of the persons, used language of the persons, utterances of the persons, speaking time of the persons, non-verbal speech features of the persons, emotional states of the persons, speech overlap or turn-taking frequency of the conversation, gestures of the persons, identities of the persons, blinking frequency of the persons, blood flow changes of the persons.

Activities of the persons may include, e.g., drinking, sitting, or walking around.

Micro-actions may include actions such as foot tapping or yawning to name some examples.

Non-verbal speech features of the persons may include prosody, for example.

Optionally, the system 1 includes a privacy filter module 5 that could be arranged after the perception module 4 and that prevents certain information to be extracted from the sensor data or the perception information, thereby protecting the privacy of the participants in the conversation.

This system 1 may activate the privacy filter module 5, e.g., based on a user setting, on a consent dialogue with the non-user participant or based on a trigger condition, such as a keyword, which signals a conversation with higher privacy implications.

Configurable user settings may determine which information is filtered. Alternatively, an explicit request e.g. issued via a dialogue or a text input of the non-user participant, a predefined set of rules dabout the sensitivity of certain information, or an algorithm that dynamically defines the sensitivity of an information in a given situation for given participants, may determine which information to filter.

The privacy filter module 5 may modify information or remove aspects of information, e.g. storing a video signal but applying a blur filter to the faces of participants or recording voices but applying a distortion filter to mask the personal information of a speaker.

If the sensing means 2 are part of an actuated system 1, e.g., a robot, the privacy filter module 5 may control the actuators to move the sensors 2 such that they are prevented from recording sensitive data, e.g., the sensors 2 may direct a coverage area of a camera away from the non-user participant.

A speaker/listener classification module 9 uses perception data provided by the perception module 4 to determine a speaker role or a listener role to communication participants. The speaker/listener classification module 9 in particular may perform a classification assigning a speaker label indicating the speaker role or a listener label indicating a listener role for any given point in time during the conversation. The speak/listener classification module 9 may perform the classification on accumulated and optionally filtered perception data from the perception module 4.

In one implementation, the speaker/listener classification module 9 uses a rule-based decision mechanism to e.g. always assign the listener label to the person that system 1 registered as its user. The speaker/listener classification module 9 may assign the listener label to the person that system 1 registered as its user based on stored face image data of the registered user and correlating a detected face in perceived image data from the environment in the perception data detection with the stored image data of the registered user. Alternatively or additionally, the speaker/listener classification module 9 may generally assign a defined label to a detected person that a specific sensor is pointing towards. Alternatively or additionally, the speaker/listener classification module 9 recognizes at least one of a predetermined keyword in acoustic perception data, a predetermined gesture in image data included in the perception data and associated with a participant to assign this participant a respective label. Alternatively or additionally, the speaker/listener classification module 9 assigns the speaker label always to the person that speaks first in the conversation. Alternatively or additionally, the speaker/listener classification module 9 assigns the speaker label to the person that enters a room where the conversation is occurring as the last participant.

Alternatively or additionally, the speaker/listener classification module 9 uses a classification algorithm to dynamically assign the speaker and listener labels during a conversation. The speaker/listener classification module 9 may implement the classification based on a model, which uses thresholds on, e.g., determined relative speaking time, non-speaking durations, silent times for the participants. The speaker/listener classification module 9 may implement the classification based on a model, which uses a content analysis system that detects syntactic means, e.g. a determined frequency of questions in acoustic data associated with individual participants or a determined semantic dominance of individual participants. The semantic dominance of individual participants may, for example, include determining who started the current topic in the conversation first.

Alternatively or additionally, the speaker/listener classification module 9 may use a machine learning system, which is trained with annotated data from conversation in a training phase before an operational phase, to map sensor data or perception data input to a speaker role or a listener role for each participating person.

Generally, the speaker label or the listener label may include a probability value for a participant having the speaker role or the listener role.

Alternatively or additionally, the speaker/listener classification module 9 may trigger a certain actuation and or output of the system 1 via a human-machine interface (HMI) not explicitly shown in fig. 1 that explicitly asks the participants to provide the labels for their current role. Alternatively or additionally, the current listener or speaker may be required or requested to push a button on a device or in an app of the HMI to signal its listener role or speaker role.

Additionally, the system 1 might include a memory, which records which participating person was assigned which label at which previous times. The system 1 may uses the previously recorded data, e.g., to bias the classification in the speaker/listener classification module 9 towards the assigned role of the speaker role or the listener role from a previous time, e.g., a previous time step in the conversation. The speaker/listener classification module 9 may bias the classification based on the recorded data taking into account a counted number of previous assignments of the speaker role or the listener role to a participating person, based on an elapsed time since a last recorded change of assignment took place, based on a determined frequency of change of assignment of the role of speaker or listener, or similar measures, or a combination of these or similar measures.

The memory may be part of a data storage 12 of the system 1.

The speaker/listener classification module 9 may communicate the generated information, on the assigned speaker label and listener label, or statistics about the generated information to a feedback generation module 10.

The system 1 comprises a situation analysis module 7, which uses the perception data input from the perception module 4 for analyzing the current state of a conversation. The situation analysis module 7 determines which of human behaviors that are part of an active listening concept, may be possible, appropriate, effective, or beneficial in the current situation in the conversation between the participants.

The situation analysis module 7 may be implemented based on a machine learning algorithm that is pre-trained on labeled data to determine a confidence value (likelihood of occurrence) for a behavior to be appropriate.

Alternatively or additionally, the situation analysis module 7 may be implemented based on a machine learning algorithm that is pre-trained on labeled data to determine an estimated predicted contribution strength towards an effective active listening behavior.

Alternatively or additionally, the situation analysis module 7 might be implemented based on an explicit decision-making algorithm, e.g., the decision-making algorithm bases on a predetermined sets of appropriate behaviors associated with a predetermined type of situation.

Alternatively or additionally, the situation analysis module 7 may take into account a recorded history of its generated output signals, e.g., in order to achieve an increased temporal stability of a set of selected behaviors. Alternatively or additionally, the situation analysis module 7 may model or be trained on a likelihood of certain transitions between situations and appropriate behaviors. E.g., after establishing an eye contact between participants, an affirmative gesture by the participant in the listening role is predicted to be highly effective.

Alternatively or additionally, the situation analysis module 7 may take into account a history of behavior classification outputs from a behavior evaluation module 8 of the system 1, e.g., if certain behaviors have been detected in the recent past, their appropriateness or likelihood to be appropriate in the perceived current situation decreases.

The situation analysis module 7 may determine conversational situations by, e.g., analyzing speaker speech content for certain triggers like a sad story being told, a problem being described, or a joke being told.

The situation analysis module 7 may determine conversational situations by analyzing speaker prosodic features to determine certain triggers like a question being asked, a voice getting louder, calmer or breaks.

The situation analysis module 7 may determine conversational situations by determining who of the participants is currently speaking, e.g., independent of the assigned speaker role or listener role.

The situation analysis module 7 may determine conversational situations by determining current body postures of the participants, relative positions of the participants, current orientation of the participants, gaze between the participants, or changes of these parameters.

The situation analysis module 7 may determine conversational situations by determining the emotional state or changes of an emotional state of the speaker. The emotional state or changes of an emotional state of the speaker may include, e.g., determining the speaker to cry, or to smile.

The situation analysis module 7 may determine conversational situations by determining an occurrence of pauses in speaking or based on pre-determined trigger behaviors of the speaker. The pre-determined trigger behaviors of the speaker may include, e.g., behaviors such as the speaker playing with their hands, yawning, or looking to the floor.

The situation analysis module 7 may determine conversational situations by analyzing a speaker behavior in the perception data for signs of lying. The system 1 may include a specific component or module using an explicit classifier for detecting indicators for the speaker lying.

The system 1 comprises a behavior evaluation module 8 that uses features provided by the perception module 4 to evaluate a current behavior of the conversation participant currently determined to have the listening role, having assigned the listening label as the current listener, with respect to different aspects of active listening targets.

The behavior evaluation module 8 may comprise multiple individual classifiers for pre-defined behaviors that are known to positively contribute to a high speaker satisfaction in a conversation situation when shown by the listener.

The individual classifiers of the behavior evaluation module 8 may be implemented based on a machine learning algorithm that is pre-trained on labeled data for each of the behaviors. Additionally or alternatively the behavior evaluation module 8 may implement the classifiers based on an explicit decision-making algorithm that maps the occurrence of a certain set of perceived features in the perception data to a certain behavior of the listener in a conversation, e.g. utterances of a certain length with a predefined frequency pattern are considered to represent a "back-channeling" behavior of the participant with the role of the listener towards the speaker.

Additionally or alternatively, the individual classifiers of the behavior evaluation module 8 may be implemented based on at least one of perceived features from previous time steps, a recorded history of the own output data generated by the behavior evaluation module 8, and a current or past output of the classifiers inside the behavior evaluation module 8.

Optionally, the behavior evaluation module 8 activates only classifiers for those behaviors of the listener, which received a certain appropriateness score or had been classified as appropriate by the situation analysis module 7.

The individual classifiers of the behavior evaluation module 8 may classify behaviors of the listener that may include, for example detecting an eye contact, exhibiting empathic facial expressions, asking open ended questions, asking comprehension questions on details of the current topic of the conversation, paraphrasing and reflecting back what has been said by the speaker, verbalizing emotional implications of what has been said by the speaker, back-channeling (sounds to signal, 'I am listening, go ahead'), mirroring or mimicking a posture of the speaker, taking an open posture and positioning relative to the speaker, approaching the speaker, exposing empathic gestures like touching the hand or handing a handkerchief, and summarizing a speech content.

Additionally, the classifiers of the behavior evaluation module 8 may provide a score for the quality of a classified behavior, taking into account, e.g., a timing, an execution, a content selection, with respect to the classified behavior of the listener.

Optionally, the behavior evaluation module 8 may comprise classifiers for behaviors which are negatively impacting the active listening targets including, e.g., classifiers for nervous motions of the listener, a staring gaze of the listener, absent minded expressions by the listener, the listener asking "why"-type questions, the listener speaking, and the listener interrupting the speaker.

The behavior evaluation module 8 may combine the results of the activated individual classifiers and the output of the situation analysis module 7 for determining an active listening score for a given time in the conversation. The determined active listening score corresponds to or reflects a possible improvement potential of the listener with regard to the active listening concept.

The system 1 may accumulate the determined active listening score over time during the conversation.

The system 1 further comprises a speaker satisfaction module 9 that evaluates the perception data output by the perception module 4 with respect to the feelings of the participant determined as speaker by the speaker/listener classification module 6 about the conversation.

The speaker satisfaction module 9 performs a classification that quantifies at least one of a satisfaction of the speaker at the current point in time, the satisfaction with a certain part of the conversation after this part has finished, e.g., when the speaker role and the listener role are determined to change during the conversation and an overall satisfaction at the end of the conversation.

The speaker satisfaction module 9 may be implemented based on a machine learning algorithm that is pre-trained on labeled data for speaker satisfaction or based on a combination of individual classifiers for certain specific behaviors known to be related to positive or negative satisfaction of the speaker.

An individual classifier for a certain specific behavior known to be related to positive satisfaction may, e.g. include a person approaches listeners and shakes hands gratefully.

An individual classifier for a certain specific behavior known to be related to negative satisfaction may include, e.g., a person leaves room where the conversation occurred.

Alternatively or additionally, the speaker satisfaction_module 9 may be implemented based on an explicit decision-making algorithm that maps a detected occurrence of certain perception features, or of a combination perception features to a contribution to an overall score for speaker satisfaction (speaker satisfaction score). The perception features may include, e.g., detecting positive emotions, smiling, appreciative words, that contribute towards higher speaker satisfaction score. The perception features may include, e.g., emotions like anger or disappointment that contribute towards lower speaker satisfaction score.

Additionally or alternatively, the speaker satisfaction module 9 may take into account perceptual features from previous time steps or a recorded history of the output generated by the speaker satisfaction module 9.

The system 1 comprises the feedback generation module 10 that determines based on the output of the behavior evaluation module 8 and the speaker satisfaction detection module 9, if, when and how a user of the system 1 could improve their active listening behavior. Determining if, when and how the user of the system 1 can improve their active listening behavior will improve communication quality of the conversation.

The feedback generation module 10 may operate on `live' data and determine a feedback to be provided to a user directly during the conversation in an online application of the system 1.

Alternatively or additionally, the feedback generation module 10 may analyze a recorded data set of a conversation that has already terminated in an offline application of the system 1 for providing an overall set of multiple feedbacks to the user to learn from a past conversation and to adapt their behavior as listeners to improve quality of future conversations, thereby improving communication quality of conversations the user will participate in in the future.

The the feedback generation module 10 detects events that indicate a potential for an improvement of user behavior, e.g., by detecting changes in the active listening score provided by the behavior evaluation module 8 that exceed a predefined threshold, by detecting changes in the speaker satisfaction score that exceed a predefined threshold, by detecting periods exceeding a predetermined time threshold in which the active listening score is below a predetermined threshold or the speaker satisfaction score is below a predetermined threshold, or by detecting an overall speaker satisfaction at the end of a conversation that is below a predetermined threshold.

For any detected event, the feedback generation module 10 searches for potential opportunities for behaviors that could improve the communication quality around the detected event. The feedback generation module 10 may use the output of the situation analysis module 7 from a range of previous times and the current time to detect behaviors that are classified as appropriate for a certain situation in the conversation and to determine those behaviors that have not been detected by the behavior evaluation module 8 to be performed by the listener. Alternatively or additionally the feedback generation module 10 determines those behaviors that have been detected by the behavior evaluation module 8 being performed by the listener but have a low quality, which corresponds to an execution quality of the behavior being smaller than a predetermined threshold. Alternatively or additionally the feedback generation module 10 determines those behaviors of the listener that have a negative impact.

Additionally, the feedback generation module 10 may use statistics of the detected active listening behaviors over a predetermined time period and select those listening behaviors having a low occurrences as general improvement targets for the user.

Additionally, the feedback generation module 10 may explicitly detect positive events that are events with that are associated with at least one of a high speaker satisfaction score, e.g. the speaker satisfaction score exceeding a predetermined threshold, and a high active listening score, e.g. the active listening score exceeding a pre-determined threshold, to share positive episodes in the analyzed conversation with the user. This may have an affirmative effect for continuing a positive active listening behavior exhibited by the user in future conversations.

The system 1 comprises a support action selection module 11 that selects how to best provide the feedback determined and generated by the feedback generation module 10 to the user.

The support action selection module 11 may determine at least supporting action dynamically based on the output of the perception module 4 or other components.

The support action selection module 11 may determine, e.g.to only show information after the speaker finished a sentence, or using an audio message instead of a visual message to the listener when the listener is currently showing positive eye contact behavior as determined by the behavior evaluation module 8.

The support action selection module 11 may perform selection additionally taking into account parameters including, e.g., an estimated perceivability of different modalities and output channels for the user in a given conversation situation, an estimated attentional capacity to fit the complexity of a given communication content.

The output modalities may include visual modality, an acoustic modality, and a haptic modality in order to name some examples.

Alternatively, the support action selection module 11 may control the provided support based on a fixed selection, e.g. for each event, display the corresponding value of the active listening score on a screen for examination or supervision by the user.

Additionally, the support action selection module 11 may perform selection taking into account if the user did react to a previous support messages, e.g., if t the user changes their listening behavior towards the listening behavior that has been previously recommended. The support action selection module 11 performing selection taking into account if the user did react to a previous support messages may include, e.g., changing a format including at least one output parameter of the output action or a modality of the output action, if the previous selection is determined to not being successful (to be ineffective).

An output parameter may include, e.g., a volume of an acoustic output, a vibration strength of haptic (tactile) output or a color of an icon on a display screen.

The support action selection module 11 may select the support action to include transmitting any combination of the current active value of the listening score, the current active value of the speaker satisfaction score, the event detected by the feedback generation module 10, the active listening behaviors that have been identified as an opportunity to improve the communication quality by the feedback generation module 10, behaviors with a potential negative influence on active listening that have been identified by the feedback generation module 10 module, the active listening behaviors that have been identified by the situation analysis module 7 to be appropriate, all scores or detections for these behaviors by the behavior evaluation module 10, statistics about detected active listening behaviors, a lack of speaker satisfaction, and potentially information how to detect it.

The support action selection module 11 may select the support action and a format of the selected support actions to include, e.g., rendered text or other visual renderings such as images, icons, animations, plots, and graphs.

The support action selection module 11 may select the support action and a format of the selected support actions to include a speech output, e.g., including longer instructive sentences or short keywords.

The support action selection module 11 may select the support action and a format of the selected support actions to include audio signals, e.g. sounds, sound sequences or audio icons that a human can interpret intuitively or based on specific instructions at the first usage of the system or a handbook or a recorded tutorial.

Tactile cues, like vibrations, pressure patterns. Targeting different body parts and/or output devices

The support action selection module 11 may select the support action and a format of the selected support actions to include a change of environmental conditions, e.g., increasing a temperature.

The support action selection module 11 may select the support action and a format of the selected support actions to include a target motion or target behavior of an actuated device or robot, e.g., turning a display screen towards the user, a robot performing the desired active listening behavior itself, a robot looking at the user to initiate an eye-contact with the user, an actuated chair turning left or right to improve the posture of the listener towards the speaker, to change the angle of the backrest to stimulate the listener to be more active and emphatic, a mobile device that can move behind the speaker to guide the gaze of the listener towards a position of the speaker in the environment, a robotic animal displaying behavior that a human interprets as curiosity to emphasize that the listener might need to ask a question.

The support action selection module 11 may select the support action and a format of the selected support actions to include a light signal, e.g. a color change, an ON/OFF-blinking pattern, activating a specific set of lights.

The support action selection module 11 may select the support action and a format of the selected support actions to include a simulation how the situation could have evolved, e.g. in a simulated scenario with a different listening behavior of the user.

In an application scenario after a conversation has finished, the support action selection module 11 may select the support action and a format of the selected support actions also taking the form of an interactive program that allows the user to scroll through, e.g., a video recording of the conversation and to obtain information about the support action selections also in an overview display, e.g. including an annotated timeline of the conversation, where the user may 'jump' to individual support events.

The support action selection module 11 may select the support action and a format of the selected support actions including information, which output channel of the output interface device 3 ( communication interface 3) is to be used for which support action.

The system may comprise a communication interface, or provide the selected support action to an external output interface device 3, both of which output (transmit) the support action selected by the support action selection module 11 to the user via the respective selected output channel(s) in the selected output format.

The communication interface may include the output channel(s) as part of the main device of the system 1. Alternatively the output interface may include individual devices separate from the main device installed or positioned in the environment connected to the compute unit and receiving the data from the support action selection module 11 via cable link, fiber optic link, or radio link.

The radio link may include wireless local area network link (WLAN), e.g. a Wifi^{™} link, a wireless personal area network link (WPAN), e.g. a Bluetooth^{™} link, a cellular radio link, e.g. in a 3G, 3.5G, 4G or 5G - generation cellular radio system.

The output channel(s) may include any combination of actuators including display screens, loudspeakers, vibration motors, actuation motors that change the position of certain elements, lights, vaporizers or similar smell emitting devices, pressure valves, on order to name some examples.

The communication interface device 3 may include a computer, a mobile phone, a wearable device, an augmented reality/virtual reality device( e.g. AR/VR glasses), a head-up display, a tabletop robot, a mobile robot, a distributed system of output devices such as a smart home system, plug-in ear phones, and headphones.

The system 1 of fig. 1 also comprises a data storage 12, which records any combination of the sensor data, perception data or any other data output of the components of the system 1, including the sensor means 2, the perception module 4, the privacy filter 5, the speaker/listener classification module 6, the situation analysis module 7, the behavior evaluation module 8, the speaker satisfaction detection module 9, the feedback generation module 10, the support action selection module 11, the communication interface and a performance tracking module 13, and which any component of the system 1 may access both during a conversation and after the conversation terminated.

Additionally, the system 1 may include a performance tracking module 13, which acquires information including the active listening score and speaker satisfaction score, statistics and scores of different active listening behaviors over multiple conversations and provides the user with an overview of how a performance of the user changes over time or statistics about the communication quality during a particular period.

Additionally, the system 1 may have a component that determines support actions based on the perception data output by the perception module 4 that can adapt a situation in a way that improves the listeners ability to perform active listening, e.g., by providing a more relaxed atmosphere by adjusting a room temperature, by playing ambient music or sounds, by recommending a break, by opening windows for an improved air.

Additionally, the system 1 may comprise a component that takes a user feedback by the user into account in order to improve future recommendations of the system 1 by learning during operation and training on training data from real operation of the system 1.

The user might press a button of an HMI of the system 1 to dismiss a concrete recommendation for an improved listening behavior as not appropriate in the current situation. Based on the feedback from the user, the system 1 adapts to reduce the chance of giving a similar recommendation in a similar situation in the future. Alternatively, the user might select a certain listening behavior as non-preferred and the system 1 adjusts future recommendations to not include this behavior in consequence. Alternatively, the user may select during offline usage of the system 1 certain situations in which the system 1 should give or should have given a recommendation to improve the classification of speaker satisfaction by the system 1.

This might be implemented in different modules based on using online or lifelong learning machine learning methods.

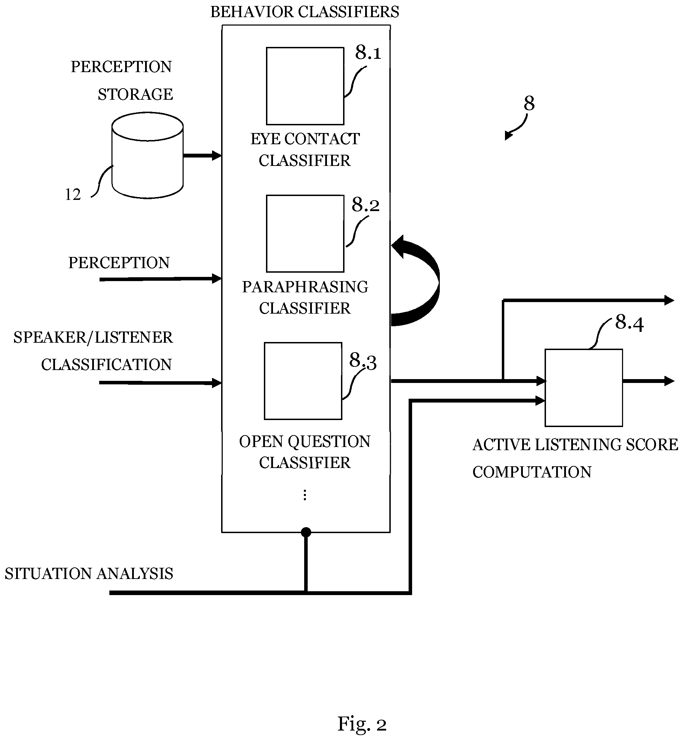

Fig. 2 presents an overview of the architecture of the behavior evaluation by an embodiment of the system 1.

The behavior evaluation module 8 obtains the perception data from the perception module 4. The input from the perception module 4 enables the behavior evaluation module based on further input from the speaker/listener classification module 6 and the situation analysis module 7 to evaluate the current behavior of the conversation participant having the listener label with respect to different aspects of active listening target, in particular the active listening behavior options (candidates) generally available, and the specific active listening behavior options suitable in the specific conversation situation.

The behavior evaluation module 8 comprises plural individual classifiers 8.1, 8.2, 8.3 for pre-defined behaviors that are known to frequently be required to be shown by a listener to contribute to or correlate with a high speaker satisfaction in a conversation situation.

The classifiers 8.1, 8.2, 8.3 maybe implemented using a machine learning algorithm trained on labeled data for each of the behaviors. The example of fig. illustrates the classifiers as an eye contact classifier 8.1, paraphrasing classifier 8.2, and an open question classifier 8.3, and further indicates that these classifiers 8.1, 8.2, 8.3 are mere examples of a large number of implemented classifiers of the behavior evaluation module 8.

Alternatively or additionally, the classifiers 8.1, 8.2, 8.3 maybe implemented using an explicit decision-making algorithm that maps the occurrence of a certain set of perceived features in the perception data including the information representing the conversation to a certain behavior correlating with an active listening behavior.

Alternatively or additionally, the classifiers 8.1, 8.2, 8.3 may use perceptual features from previous time steps during the conversation, a history of the classifier output, the current or past output of other classifiers 8.1, 8.2, 8.3 of the behavior evaluation module 8

The classifiers 8.1, 8.2, 8.3 may determine a score for the quality of a certain behavior identified in the information representing the conversation, taking into account, e.g. timing, execution or content selection.

The behavior evaluation module 8 further comprises an active listening score computation module 8.4, that computes a current active listening score value for the current conversation situation based on the outputs of the plural classifiers 8.1, 8.2, 8.3, ..8.n, and based on the analyzed conversation situation provided by the situation analysis module 7.

The behavior evaluation module 8 provides the information on the behaviors in the current conversation situation generated by the classifiers 8.1, 8.2, 8.3, ... to the feedback generation module 10.

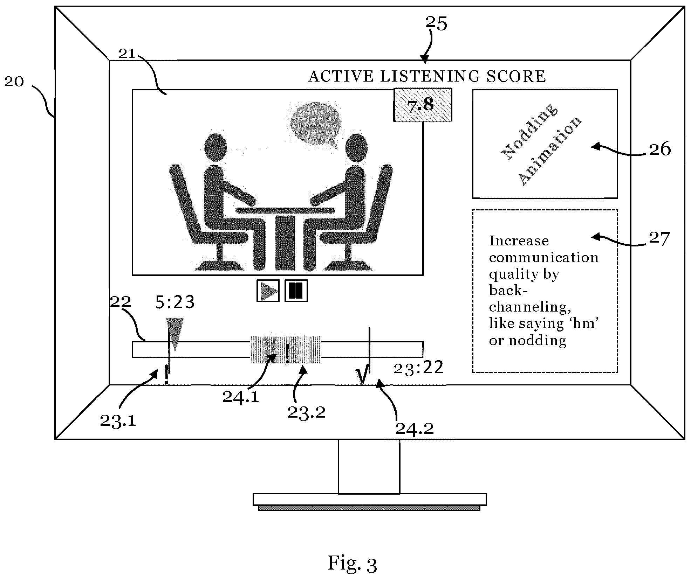

Fig. 3 presents an example of a screen 20 of the interface illustrating an aspect of the architecture of an embodiment of the system 1 in an offline training application.

The communication interface or the output interface device 3 may include a computer with a monitor running a software application for displaying the screen 20 implementing a graphical user interface (GUI) configured to train active listening based on the described system 1.

Application is used after a conversation has occurred in order to teach the user how to improve listening behavior for future conversations. This represents as an advantageous offline application of the disclosed system 1.

The user can watch the recorded conversation as a video/audio stream 21 in a part of the screen 20 and jump to different time points using a time bar 22 displayed by the GUI on the screen 20.

The time bar 22 may include highlighted sections 23 associated with moments 23.1 or periods 23.2 in the recorded conversation for which the system 1 determines support to be available, e.g. an identified potential improvement marked with an exclamation sign "!" 24.1 and a successful behavior marked by a hook sign"√" 24.2.

The system 1 displays the active listening score in a box-like section 25 on the screen 20 with its current computed value "7.8" for the current time of the reproduced video/audio stream 21.

If the user jumps to a specific moment 23.1 or period 23.2 for which the system 1 offers a support, the application will provide a suggestion on how the user could have improved their listening behavior in the specific moment 23.1, using an nodding animation 26 and a textual description 27 of the suggested listening behavior.

The system 1 may be advantageously applied in offline usage scenarios. Ina first scenario, a doctor and a patient have a medical conversation. The patient may be a child.

The system 1 acquires sensor data on the conversation. The system 1 may apply applying privacy preserving mechanisms, e.g., using a privacy filter 5 on the acquired sensor data due ensuring confidentiality of the conversation.

The system 1 performs speaker/listener detection, resulting in the respective roles of the listener and the speaker being assigned to the participating persons, and switched during the conversation when the system 1 detects a change in the roles.

The sensor data including the information representing the conversation, in particular the information from the listener is analyzed and an active listening model determines the active listening behavior as well as the active listening score.

The sensor data including the information representing the conversation, in particular the information from the speaker is analyzed and the system 1 determines the speaker satisfaction.

The sensor data, the role assignment, the active listening behaviors (active listening features), the computed active listening score, and the determined speaker satisfaction are stored into a database, in particular the data storage 12.

The child might explain the own situation vague, maybe omitting some details since they are embarrassing or seem to be unimportant. The child may potentially bend the truth or even lying.

The doctor may be unexperienced and may not notice verbal or non-verbal signals indication where to ask for more details or background or asking directly for specific and important information about the child's situation.

As a consequence the child might leave the doctor, feeling uncomfortable due to the insecurity if lying about information is relevant or even critical. The child might leave the doctor, feeling uncomfortable due to the way the topic perceived as embarrassing from the child's point of view and related information was discussed.

In such situation, the system enables the doctor to learn from the stored and analyzed conversation where the course of the conversation could be improved from their side. The system 1 reproduces the conversation and based on the computed active listening score, and the determined speaker satisfaction, an offline analysis of the most critical situations on the display screen 20 indicates when during the conversation doctor could have acted differently and acted with improved active listening features providing support using different modalities: the system 1 may in particular provide suggestions to the doctor via text instructions and visual pointing; the system 1 may use audio explanations, audio instructions and visual pointing of features The system may even indicate a simulation on screen of how the situation might have evolved differently if the doctor had shown a different active listening behavior and used additional listening behaviors exceeding those already shown.

In these offline application scenarios, the system 1 can express the determined low speaker satisfaction and pointing the doctor to the specific situations during the recorded conversation in which the doctor can recognize and learn why the speaker, here the child, was not satisfied with the conversation.

The system 1 points on the display screen 20 in the reproduced situation to expressions of the child indicating emptions including signs of, e.g., fear or insecurity.

System points to situations where the listener had a lack of facial expressions showing empathy

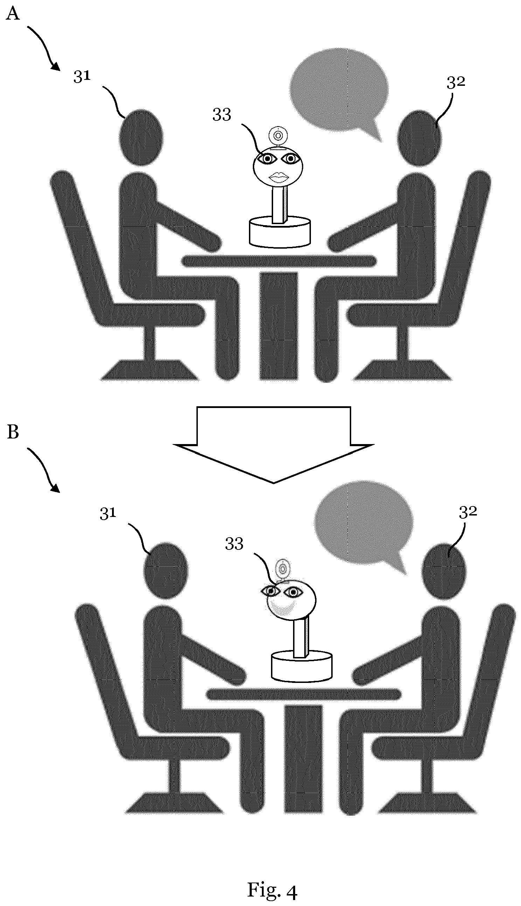

Fig. 4 illustrates an example design of an output interface device 3 of the system 1 in an embodiment in an online feedback application scenario.

A robotic device 33 comprising the system 1 according to an embodiment is adapted to support a first person 31 representing the user in improving their listening behavior during a conversation with a second person 32.

The first person 31 (user) is listening to the second person 32 their conversation partner in the upper part A of fig. 4.

The system 1 classifies the first person 31 as the listener and the second person 32 as the speaker in the conversation. The system 1 determines that a satisfaction of the speaker decreases and detects that the listener and simultaneously user is not performing all active listening behaviors as potential behaviors that the system 1 determines as appropriate in the current situation of the conversation.

The system 1 determines that the listener might perform an additional behavior in order to improve the satisfaction level of the speaker in the conversation, in particular the current situation. The system 1 determines as the additional behavior that to inform the user and listener that the additional behavior of showing empathy, for example by smiling, could improve the communication quality of the conversation monitored by the system 1. The system 1 selects for the robot device 33 a supporting action including the robot device 33 may turn towards the user and change a facial expression of the robot device 33 to a smile will prompt the user to smile in turn and thereby improve the satisfaction of the speaker due to exhibiting improved communication skills. The system 1 controls the robot device 33 therefore to execute the selected supportive action as illustrated in the lower part B of fig. 4.

The user understands the message and will themselves smile towards the Speaker A specific online application scenario of the system 1 may include a conversation of a lawyer and a client meeting for the first time. The clients has a specific legal issue that they want the lawyer to take care of.

During the conversation, the lawyer is reading documents about the case brought by the client, while the client is describing verbally the problem and a desired outcome.

Most of the relevant facts about the case are already represented in the document that has been send to the lawyer beforehand.

Without using the system 1, the lawyer sometimes looks up and nods and is listening, but his eyes and posture turn back to the documents often. After finishing reading the documents, the lawyer uses a pause in the client's speech to describe the most promising strategy and likely outcomes. When the client leaves the law office, he will have a feeling that the lawyer might be confident to reach a solution corresponding to the desired outcome, however, the client might not trust a proposal of the lawyer due to feeling a perceived lack of interest of the lawyer to the specific details important for the client. The client might approach an alternative lawyer for the case.

Using the system 1 in an online application scenario during the conversation, the lawyer is wearing earphones that are connected as an output interface device 3 to the system 1. The meeting room has a camera installed at the ceiling. The camera provides sensor data, the 1 uses to generate information representing the conversation. In this specific scenario, the system 1 assigns the listener role to the lawyer as the user of the system 1 by default.

After the client has been speaking for some time, the system 1 detects prosodic features in the speech signal of the client that are correlated with a deteriorating satisfaction compared to data from the beginning of the meeting.

The situation analysis module 7 computes that the client is currently talking about an emotional situation and determines that appropriate active listening behaviors would be to establish eye-contact and make an empathic statement.

The behavior evaluation module 8 determines, that the speaker (client) did establish eye-contact at a specific time during the conversation, but did not make any statements in the last minute, and therefore classifies the current active listening score as having a low value at this point during the conversation. The feedback generation module 10 determines based on the current active listening score value that the system 1 should recommend the listener (lawyer) to make an empathic statement.

The system 1 has a pre-determined setting for such output situations, and therefore the lawyer will receive a recorded vocal statement via the earphones stating "show some empathy". This will trigger the lawyer to say "I see that you felt as if you did not have another chance due to the stressful situation". After finishing reading the documents, the lawyer uses a pause in the client's speech to describe the most promising strategy and likely outcomes.

When leaving the meeting with the lawyer, the client has the impression that the lawyer is confident to reach a good solution. In addition, the client feels that the lawyer understood the specifics of the situation and that the proposed solution is tailored to their individual case. As they are very satisfied with the meeting, they will hire the lawyer for their case.

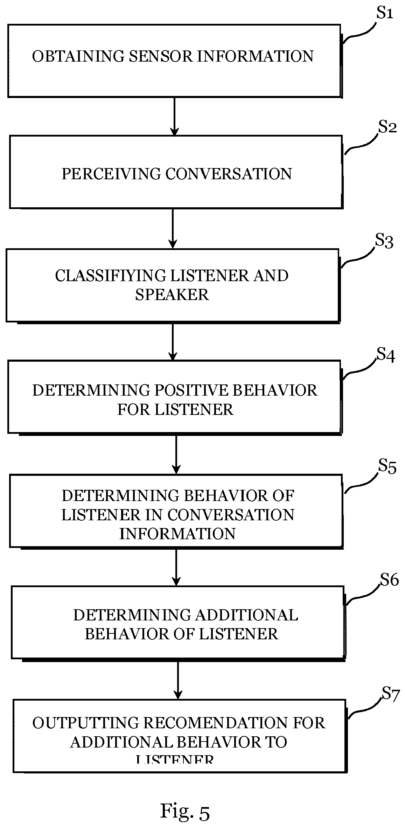

Fig. 5 shows a schematic flow diagram illustrating steps of a computer-implemented method according to an embodiment.

The computer-implemented method addresses the target of assisting a person in improving its conversation skills. The method starts with a step S1 of obtaining sensor information. The obtained sensor information includes visual information on the participants of the conversation and acoustic information, in particular speech information of the conversation.

In step S2, the method proceeds with perceiving, by a perception module, a conversation of the person and at least one other person by generating information on the conversation based on acquired sensor information.

Step S3, includes classifying, by a processing module, the assisted person and the at least one other person into at least one listener and a speaker based on the generated information on the conversation.

In step S4 the processing module determines, which behavior of the person classified as the listener correlates with a positive quality of the conversation by evaluating the current conversation situation based on the generated information on the conversation.

The processing module determines in step S5, which behavior the person classified as the listener is performing based on the generated information on the conversation,

In step S6 the processing module determines at least one additional behavior for execution by the person classified as the listener that improve the quality of the conversation based on the generated information on the conversation.

The processing module then controls, in step S7, an output via an interface to the person classified as the listener of a recommendation for an improved communication generated based on the determined at least one additional behavior.

All steps which are performed by the various entities described in the present disclosure as well as the functionalities described to be performed by the various entities are intended to mean that the respective entity is adapted to or configured to perform the respective steps and functionalities.

In the claims as well as in the description the word "comprising" does not exclude the presence of other elements or steps. The indefinite article "a" or "an" does not exclude a plurality.

A single element or other unit may fulfill the functions of several entities or items recited in the claims. The mere fact that different dependent claims recite certain measures and features of the control circuit does not exclude that a combination of these measures and features cannot combined in an advantageous implementation.

## Claims

1. System for assisting a person in improving its conversation skills, the system comprising:
a perception module configured to perceive a conversation between the person and at least one other person by generating information representing the conversation based on acquired sensor information;
a processing module configured to classify the assisted person and the at least one other person into at least one listener and a speaker based on the generated information,
to determine which potential behavior of the person classified as the listener correlates with a positive quality of the conversation by evaluating the current conversation situation based on the generated information,
to determine which actual behavior the person classified as the listener is performing based on the generated information,
to determine at least one additional behavior for execution by the person classified as the listener that improves the quality of the conversation based on the generated information;
an interface configured to output a recommendation for an improved communication behavior to the person classified as the listener generated based on the determined at least one additional behavior.

2. System according to claim 1, wherein
the perception module is configured to generate the information representing the conversation based on the perceived complete conversation of the person and the at least one other person;
the processing module is configured to analyze the information offline, and
the interface is configured to output the recommendation for an improved communication behavior to the person classified as the listener generated based on the determined at least one additional behavior for the complete conversation as training information.

3. System according to claim 1, wherein
the perception module is configured to generate the information representing the conversation based on the perceived conversation of the person and at least one other person while the conversation is ongoing;
the processing module is configured to analyze the information online while the conversation is ongoing, and
the interface is configured to output the recommendation for an improved communication behavior to the person classified as the listener generated based on the determined at least one additional behavior as an online guidance while the conversation is still ongoing.

4. System according to any one of the preceding claims, wherein
the processing module is configured to determine based on the information representing the conversation a satisfaction measure for the person classified as the speaker with the conversation.

5. System according to any one of the preceding claims, wherein
the processing module is configured to dynamically reclassify the person and the at least one other person into a t least one new listener and a new speaker based on the generated information representing the conversation during the conversation.

6. System according claim 5, wherein
the processing module is configured to control the interface output the recommendation for an improved communication behavior to the person classified as the at least one new listener after dynamically reclassifying the person and the at least one other person into at least one new listener and a new speaker.

7. System according to any one of the preceding claims, wherein
the system comprises a privacy filter configured to filter at least one of the acquired sensor information, the generated information representing the conversation by applying at least one predetermined confidentiality rule on the at least one of the acquired sensor information.

8. Computer-implemented method for assisting a person in improving its conversation skills, the method comprising:
perceiving, by a perception module, a conversation of the person and at least one other person by generating information representing the conversation based on acquired sensor information;
classifying, by a processing module, the assisted person and the at least one other person into at least one listener and a speaker based on the generated information;
determining, by the processing module, which potential behavior of the person classified as the listener correlates with a positive quality of the conversation by evaluating the current conversation situation based on the generated information;
determining, by the processing module, which actual behavior the person classified as the listener is performing based on the generated information;
determining, by the processing module, at least one additional behavior for execution by the person classified as the listener that improve the quality of the conversation based on the generated information; and
controlling, by the processing module, an output via an interface to the person classified as the listener of a recommendation for an improved communication generated based on the determined at least one additional behavior.

9. Computer program comprising instructions, which, when a computer or signal processor executes the program, cause the computer or signal processor to carry out the method of claim 8.
